Europäisches Patentamt

European Patent Office

Office européen des brevets

(11) Publication number: **0 378 691**
**A1**

# EUROPEAN PATENT APPLICATION
## published in accordance with Art. 158(3) EPC

(21) Application number: 89906461.2

(51) Int. Cl.⁵: **A61B 17/36**

(22) Date of filing: 06.06.89

(86) International application number:
**PCT/JP89/00572**

(87) International publication number:
**WO 89/11832 (14.12.89 89/29)**

(30) Priority: 06.06.88 JP 74911/88 U

(43) Date of publication of application:
**25.07.90 Bulletin 90/30**

(84) Designated Contracting States:
**DE FR GB IT NL**

(71) Applicant: **SUMITOMO ELECTRIC INDUSTRIES, LTD**
**5-33, Kitahama 4-chome Chuo-ku**
**Osaka 541(JP)**

(72) Inventor: **SOGAWA, Ichiro Osaka Works of Sumitomo Electr.**
**Ind., Ltd. 1-3, Shimaya 1-chome**
**Konohana-ku Osaka-shi, Osaka 554(JP)**
Inventor: **NIWA. Shin-ichiro Osaka Works of Sumitomo Electr.**
**Ind., Ltd. 1-3, Shimaya 1-chome**
**Konohana-ku Osaka-shi, Osaka 554(JP)**
Inventor: **YOTSUYA, Koro Osaka Works of Sumitomo Electr.**
**Ind., Ltd. 1-3, Shimaya 1-chome**
**Konohana-ku Osaka-shi, Osaka 554(JP)**
Inventor: **UEMIYA, Takafumi Osaka Works of Sumitomo Electr.**
**Ind., Ltd. 1-3, Shimaya 1-chome**
**Konohana-ku Osaka-shi, Osaka 554(JP)**
Inventor: **KANAZAWA, Shin-ichi Osaka Works of Sumitomo Electr**
**Ind., Ltd. 1-3, Shimaya 1-chome**
**Konohana-ku, Osaka-shi, Osaka 554(JP)**

(74) Representative: **Lehn, Werner, Dipl.-Ing. et al**
**Hoffmann, Eitle & Partner Patentanwälte**
**Arabellastrasse 4**
**D-8000 München 81(DE)**

(54) **LASER-GUIDE FIBER.**

(57) A laser-guide fiber is provided with a vibration member at its tip. This member vibrates the fiber tip peipendicularly to the fiber axis, and correspondingly the beam spot reciprocates periodically. This en-

ables irradiation of a large area with a laser beam.

# F i g . 1

SPECIFICATION

LASER BEAM GUIDING FIBER

[FIELD OF THE INVENTION]

The present invention relates to a laser beam guiding fiber, and in particular, relates to a laser beam guiding fiber for the use in, for example, a catheter for lasing in which laser beam irradiation cures a lesion portion.

[BACKGROUND OF THE INVENTION]

Catheters for the use of lasing have been developed which removes a lesion portion such as a thrombus in a blood vessel, which a practitioner cannot directly approach, by means of irradiating with a laser beam and heat-vaporizing the portion. In such the lasing catheter, an optical fiber (a laser beam guiding fiber) is used to guide the laser beam to a tip of the catheter.

With the use of a conventional laser beam guiding fiber, since radiated light is in the form of thin beam in order to increase an irradiated energy density, there exists a following problem, for example, in the case of treating the lesion portion, such as an obstructed portion in the blood vessel in which it is difficult for the tip of the catheter to move widely.

That is, when the lesion portion would be irradiated with the laser beam widely in the case where such the lesion portion extends widely, the tip of the laser beam guiding fiber should be apart from the lesion portion, so that the irradiating energy density of the laser beam would be reduced. Therefore, it is necessary to increase an amount of the laser beam energy which enters the laser beam guiding fiber in order to irradiate the lesion portion with a sufficient amount of energy. However, when the amount of the laser beam energy is increased, the laser beam guiding fiber tends to be destroyed by the energy of the laser beam.

[OBJECT OF THE INVENTION]

The present invention has been made by taking account of the above problem. It is an object of the present invention to provide a laser beam guiding fiber which can guide a large amount of laser radiating energy and irradiate all over the lesion portion with the laser beam.

[DISCLOSURE OF THE INVENTION]

A laser beam guiding fiber according to the present invention is characterized in that an oscillating member is disposed at a tip region of an optical fiber, which oscillating member oscillates the tip of the optical fiber along a direction which intersects an axis of the optical fiber.

Then, the laser beam guiding fiber according to the present invention can achieve a wide laser irradiating range since the tip of the optical fiber oscillates by means of the oscillating member along the direction which intersects the axis of the fiber and the irradiated position with the laser beam reciprocates periodically with the oscillation.

The laser beam guiding fiber according to the present invention will be hereinafter described in detail with reference to the accompanying drawings which show the embodiments of the present invention.

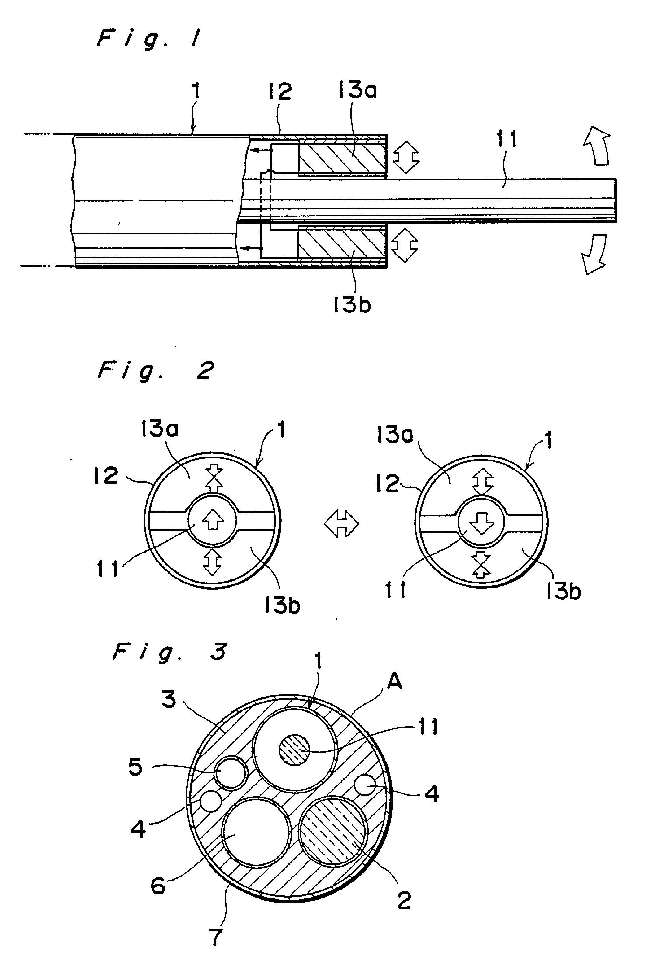

Referring to the drawings, Figure 1 shows a sectional view of one embodiment of the tip portion of the laser beam guiding fiber according to the present invention,

Figure 2 shows a front view of the above embodiment showing the oscillation mode of the optical fiber,

Figure 3 shows a sectional view of one embodiment of the catheter for the use of the lasing, in which the laser beam guiding fiber is incorporated,

Figure 4 shows a sectional view of a tip portion of another embodiment of the laser beam guiding fiber according to the present invention, and

Figure 5 shows a method in which the laser beam guiding fiber according to the present invention is used.

The laser beam guiding fiber 1 of Figure 1 comprises an optical fiber 11, a sheath 12 in the form of an elongated cylinder which receives the optical fiber 11 and piezoelectric oscillators 13a and 13b as the oscillating members. Only the tip of the optical fiber 11 projects out of the sheath 12, and the projected portion is sandwiched between the piezoelectric oscillators 13a and 13b which are disposed inside of the tip portion of the sheath 12.

As the optical fiber 11, any type of the optical fiber can be selected from the known optical fibers such as a silica optical fiber, a silica-core plastic-cladding optical fiber, a plastic optical fiber and so on, depending on the type of the laser to be used. In particular, when excimer laser is used for the lasing, the pure silica-core optical fiber is preferably used since it can guide the excimer laser beam with a high energy density and a low loss.

Each of the piezoelectric oscillators 13a and 13b is formed, as shown in Figure 2, such that it is fixed into the cylindrical space between the optical fiber 11 and the sheath 12. The oscillating direction of the piezoelectric oscillators is such that the distance between the inner circle which contacts the optical fiber 11 and the outer circle which contacts the sheath 12 is changed when the voltage is applied. In addition, the two piezoelectric oscillators 13a and 13b are, as shown with the arrows in Figure 2, such that the phases of change in their thickness are reversed in order to smoothen the oscillation of the optical fiber sandwiched on its both sides. Thus, there are a number of ways to reverse the phases of change in the thickness of the two oscillators 13a and 13b. For example, the piezoelectric oscillators 13a and 13b which are made so as to change along the same direction one another are connected, as shown in Figure 1, such that the phases of the applied voltage are reversed, whereby the phases of the thickness change of both oscillators are reversed.

As the piezoelectric oscillator, any known piezoelectric oscillator can be used, for example a ceramic oscillator such as a one-component system (for example $BaTiO_3$), a two-component system (for example $PbTiO_3-PbZrO_3$) and a three-component system (for example $Pb \cdot (Ti,Zr) \cdot O_3$ alloyed with a third component) and a crystal oscillator such as a quartz oscillator.

The oscillating member used in the present invention is not limited to such the piezoelectric oscillator as described above, and other type of the oscillator can be used, for example, a oscillator in which an oscillating magnetic field is induced with an electromagnetic means such as a coil so that a magnetic material can be oscillated.

In the laser beam guiding fiber 1 as shown in Figure 1, the laser beam transmitted through the optical fiber 11 is radiated from the tip of the fiber 11. Since the tip oscillates due to the piezoelectric oscillators 13a and 13b along the direction which intersects the axis of the fiber and the irradiated position with the laser beam reciprocates periodically with the oscillation of the fiber tip, the irradiated range with the laser beam can be enlarged.

The laser beam guiding fiber 1 can be applied to a catheter A for the lasing, for example, as shown in Figure 3. The lasing catheter A shown in Figure 3 comprises, in the elongated cylindrical sheath 7, the laser beam guiding fiber 1 described above, an imaging fiber 2 for endoscope and spectral observation, a tube 5 to supply expanding gas to a balloon (not shown), for example, in order to fix the tip and a tube 6 for removing blood or dosing a medicine. In a space among the fibers 1 and 2 and the tubes 5 and 6, filled is a transparent medium 3 comprising a material transparent against visible light such as a transparent resin and a multi-component glass rich in flexibility. This transparent medium 3 can be also used as a light guide for illumination at the tip portion. Further, a bore 4 may be provided through which a controlling wire extends in order to control the tip portion of the catheter.

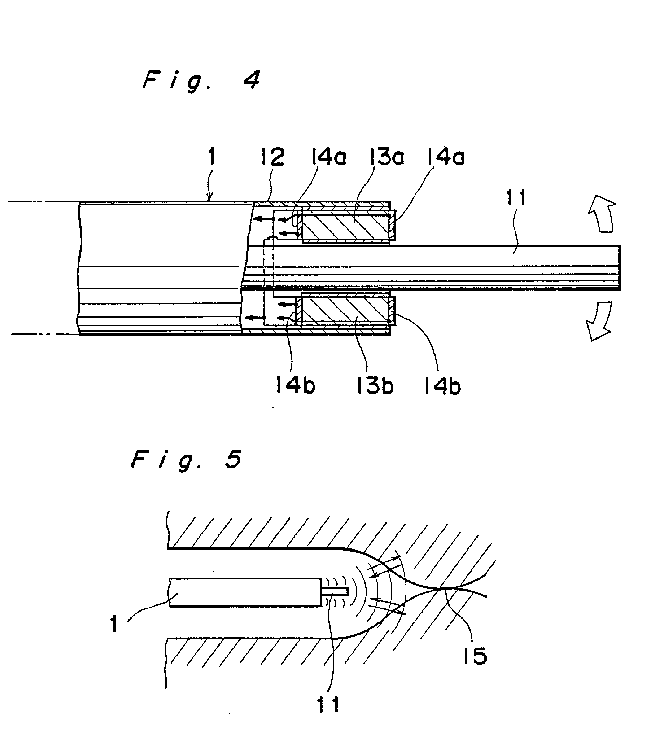

Another embodiment of the laser beam guiding fiber according to the present invention is shown in Figure 4. In this embodiment, the oscillating members act as supersonic oscillators and also supersonic detectors. Oscillating electrodes 14a and 14b excite the oscillating members along the axis of the fiber and radiate supersonic wave, and also detect supersonic wave along the axis of the fiber.

A method in which the laser beam guiding fiber described above is used will be described with reference to Figure 5.

The tip of the laser beam guiding fiber is guided to the vicinity of an obstructed portion 15 in the blood vessel and the obstructed portion is irradiated with the laser beam through the fiber to be removed through vaporizing. At the same time, the oscillating members radiate supersonic wave and the reflected wave from the obstructed portion and/or the wall of the blood vessel is detected with the same oscillating members. In this way, it is possible to evaluate an extent of the vaporization with the laser beam irradiation. Also, with the reflection of supersonic wave, it is possible to measure the distance between the tip of the laser beam guiding fiber and the obstructed portion. Further, scanning of the supersonic beam in the blood vessel is possible, for example, by constituting the oscillating members with a plurality of oscillators and lagging the phases of their exciting. In such the case, the shape of the obstructed portion can be also determined. Since the supersonic wave not only reflects at the surface of tissue but also enters the inside of the tissue, it is also possible to grasp the inner configuration of the obstructed portion. In addition, by measuring Doppler shift of the wave reflected with hemocyte in blood, it is possible to determine a velocity of bloodstream, so that initiation of re-flowing of blood resulted from removal of the obstructed portion by the laser irradiation.

The laser beam guiding fiber according to the present invention is not limited to the embodiments described above and a number of modifications in design are possible within the scope of the following claims of the present invention. For example, although two piezoelectric oscillators are provided in the embodiment described above, the number of the oscillating member may be one. Further, the oscillating member is not necessarily fixed to the optical fiber. The oscillation of the fiber tip can be changed by inserting or drawing the fiber optionally in order to change the exciting position.

With the laser beam guiding fiber according to the present invention, the tip of the optical fiber oscillates along the direction which intersects the axis of the fiber and thereby the irradiated position with the laser beam reciprocates periodically with the above oscillation, so that the laser irradiating range, as a whole, can be enlarged. Therefore, since it is possible to irradiate all over the lesion portion with the laser beam while the tip of the laser beam guiding fiber can be approach sufficiently the lesion portion, safe curing over the wide range is possible at once.

What is claimed is:

1. A laser beam guiding fiber comprising an optical fiber and an oscillating member which oscillates a tip of the optical fiber along a direction which intersects an axis of the optical fiber.

2. The laser beam guiding fiber according to claim 1 wherein the oscillating member is a supersonic oscillator and also a supersonic detector which oscillates the tip of the optical fiber along the direction which intersects the axis of the optical fiber, and which radiates supersonic wave along the axis of the optical fiber and detects the supersonic wave reflected from a object which exists along the axis of the optical fiber.

Fig. 1

Fig. 2

Fig. 3

Fig. 4

Fig. 5

# INTERNATIONAL SEARCH REPORT

International Application No  PCT/JP89/00572

## I. CLASSIFICATION OF SUBJECT MATTER (if several classification symbols apply, indicate all) 6

According to International Patent Classification (IPC) or to both National Classification and IPC

Int. Cl⁴　　A61B17/36

## II. FIELDS SEARCHED

### Minimum Documentation Searched 7

| Classification System | Classification Symbols |
|---|---|
| IPC | A61B17/36, A61N5/06 |

### Documentation Searched other than Minimum Documentation to the Extent that such Documents are Included in the Fields Searched 8

| | |
|---|---|
| Jitsuyo Shinan Koho | 1968 - 1989 |
| Kokai Jitsuyo Shinan Koho | 1971 - 1989 |

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| Y | JP, A, 59-42502 (Matsushita Electric Ind. Co., Ltd.) 9 March 1984 (09. 03. 84) (Family : none) | 1 |
| Y | JP, A, 62-299915 (Matsushita Electric Ind. Co., Ltd.) 26 December 1987 (26. 12. 87) (Family : none) | 1 |
| Y | JP, A, 59-46952 (Hitachi, Ltd.) 16 March 1984 (16. 03. 84) (Family : none) | 2 |
| X | JP, A, 57-37434 (Olympus Optical Co., Ltd.) 1 March 1982 (01. 03. 82) (Family : none) | 1 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| August 8, 1989 (08. 08. 89) | August 28, 1989 (28. 08. 89) |
| International Searching Authority | Signature of Authorized Officer |
| Japanese Patent Office | |

Form PCT/ISA/210 (second sheet) (January 1985)